(19) Europäisches Patentamt
European Patent Office
Office européen des brevets

(11) Veröffentlichungsnummer: **0 123 763**
**A2**

# (12) EUROPÄISCHE PATENTANMELDUNG

(21) Anmeldenummer: **83810215.0**

(22) Anmeldetag: **20.05.83**

(51) Int. Cl.³: **D 21 D 3/00**, D 21 H 3/02, C 07 C 143/72

(30) Priorität: **30.03.83 CH 1757/83**

(43) Veröffentlichungstag der Anmeldung: **07.11.84 Patentblatt 84/45**

(84) Benannte Vertragsstaaten: **AT BE CH DE FR GB IT LI NL SE**

(71) Anmelder: **CIBA-GEIGY AG, Postfach, CH-4002 Basel (CH)**

(72) Erfinder: **Bernheim, Michael, Dr., Angensteinerweg 15, CH-4144 Arlesheim (CH)**
Erfinder: **Meindl, Hubert, Dr., Kornfeldstrasse 77, CH-4125 Riehen (CH)**
Erfinder: **Rohringer, Peter, Sechsjuchartenstrasse 1, CH-4124 Schönenbuch (CH)**
Erfinder: **Wegmüller, Hans, Dr., Kornfeldstrasse 18, CH-4125 Riehen (CH)**
Erfinder: **Werthemann, Dieter, Dr., Scherkesselweg 15, CH-4052 Basel (CH)**

(54) **Verfahren zum Leimen von Papier mit anionischen, hydrophoben Leimungsmitteln und kationischen Retentionsmitteln.**

(57) Leimungsmittel, welche neue Verbindungen darstellen und mindestens eine anionische oder acide, gegebenenfalls in Salzform vorliegende Gruppe und mindestens zwei hydrophobe Substituenten aufweisen, wobei mindestens zwei der nächst benachbarten hydrophoben Substituenten miteinander über ein Verbindungsglied verknüpft sind, das mindestens 1 Kohlenstoffatom und 2 Heteroatome, wobei mindestens 1 Schwefelatom als Heteroatom vorhanden ist, enthält, insbesondere Sulfimide oder Bisulfimide aus Fettsäurederivaten, eignen sich besonders gut dazu, zusammen mit handelsüblichen Retentionsmitteln in einem Verfahren zum Leimen von Papier oder Karton eingesetzt zu werden.

EP 0 123 763 A2

CIBA-GEIGY AG
Basel (Schweiz)

1-14370/+

# Verfahren zum Leimen von Papier mit anionischen, hydrophoben Leimungsmitteln und kationischen Retentionsmitteln

Der vorliegenden Erfindung liegt die Aufgabe zu Grunde, dem Papierhersteller leicht zugängliche und auf einfache Art erhältliche Leimungsmittel zur Verfügung zu stellen, die unter Mitverwendung von herkömmlichen, kationischen Retentionsmitteln in neuartiger Kombination geeignet sind, eine gute Leimung bei der Papierherstellung aus Faserstoffsuspensionen zu bewirken.

Diese Aufgabe wird erfindungsgemäss so gelöst, dass bei der Papierherstellung unter Mitverwendung von polymeren, kationischen Retentionsmitteln, Leimungsmittel eingesetzt werden, die mindestens zwei langkettige, hydrophobe Substituenten und mindestens eine anionische oder acide (saure), gegebenenfalls in Salzform vorliegende Gruppe aufweisen.

Gegenstand der vorliegenden Erfindung ist somit ein Verfahren zum Leimen von Papier oder Karton, das dadurch gekennzeichnet ist, dass man zu wässrigen, cellulosehaltigen, gegebenenfalls füllmittelhaltigen Faserstoffsuspensionen mindestens

(A) ein Leimungsmittel, welches mindestens eine anionische oder acide, gegebenenfalls in Salzform vorliegende Gruppe und mindestens zwei hydrophobe Substituenten mit jeweils mindestens 5 Kohlenstoffatomen enthält, wobei mindestens einer der hydrophoben Substituenten mindestens 8, vorzugsweise 8 bis 22, insbesondere 16 bis 20 Kohlenstoffatome enthält und mindestens zwei der nächst benachbarten hydrophoben Substituenten miteinander über ein Verbindungsglied verknüpft sind, das mindestens 1 Kohlenstoffatom

und 2 Heteroatome, wobei mindestens ein Schwefelatom als Heteroatom vorhanden ist, enthält, und

(B) ein polymeres, kationisches Retentionsmittel
in beliebiger Reihenfolge oder gleichzeitig hinzugibt.

Weitere Erfindungsgegenstände bilden

- die wässrigen Zusammensetzungen zur Durchführung des Papierleimungsverfahrens, die, sofern das Leimungsmittel (A) und das Retentionsmittel (B) in beliebiger Reihenfolge zur Faserstoffsuspension separat gegeben werden, neben fakultativen üblichen Zusätzen das Leimungsmittel (A) allein, das mindestens teilweise in Form von Salzen vorliegt, oder, sofern das Leimungsmittel (A) und das Retentionsmittel (B) der Faserstoffsuspension gleichzeitig zugegeben werden, sowohl das gegebenenfalls mindestens teilweise in Salzform vorliegende Leimungsmittel (A) als auch das Retentionsmittel (B) neben fakultativen, üblichen Zusätzen enthalten,
- das (der) nach dem erfindungsgemässen Verfahren geleimte Papier oder Karton und
- die Verwendung des Leimungsmittels (A) der angegebenen Art zum Leimen von Papier oder Karton.

Die angegebenen Leimungsmittel (A) stellen neue Verbindungen dar, welche zusammen mit dem Verfahren zu deren Herstellung ebenfalls weitere Gegenstände der vorliegenden Erfindung bilden.

Als wesentliches Merkmal weisen die erfindungsgemäss verwendeten Leimungsmittel (A) im allgemeinen 1 oder 2 potentiell anionische Gruppen auf, die in der Regel als acide Imingruppen (-NH) vorliegen. Diese potentiell anionischen Gruppen können in wässrigem Medium bei den üblicherweise bei der Papierherstellung vorliegenden pH-Werten der Faserstoffsuspensionen Anionen bilden. Unter den genannten Bedingungen können andererseits auch die kationischen Retentionsmittel (B) Kationen bilden. Die Fähigkeit der Leimungsmittel und der Retentionsmittel, bei den Bedingungen der Papierherstellung Anionen bzw.

Kationen zu bilden, kann auch als anionaktiv bzw. kationaktiv bezeichnet werden. Somit können die anionischen Leimungsmittel und die kationischen Retentionsmittel auch anionaktive Leimungsmittel bzw. kationaktive Retentionsmittel genannt werden.

Als weiteres kennzeichnendes Merkmal weisen die Leimungsmittel (A) 2 bis 5, vorzugsweise 2 oder 3 hydrophobe, nur aus Kohlenstoff- und Wasserstoffatomen bestehende Substituenten mit mindestens 5, vor allem 6 bis 22, vorzugsweise 8 bis 22, insbesondere 16 bis 20 Kohlenstoffatomen, z.B. $C_5$-$C_{12}$-Cycloalkyl- oder $C_6$-$C_{10}$-Aryl-, -Alkaryl- oder -Aralkylreste auf. Bevorzugte hydrophobe Substituenten sind indessen unsubstituiertes oder mit $C_1$-$C_4$-Alkyl substituiertes Phenyl, insbesondere Alkyl- oder Alkenylreste, welche sich in der Regel von ungesättigten oder gesättigten Fettsäuren, Fettalkoholen oder Fettaminen mit mindestens 6, vorzugsweise 8 bis 22, insbesondere 16 bis 20 Kohlenstoffatomen ableiten.

Die Verknüpfungsart, mit welcher diese hydrophoben Substituenten miteinander verbunden sind, bildet ein weiteres Kennzeichen der Leimungsmittel (A). Die zweiwertigen Verbindungsglieder, über welche mindestens zwei der nächst benachbarten hydrophoben Substituenten verknüpft sind, weisen nämlich vorzugsweise 1 bis 15, insbesondere 1 bis 8 Kohlenstoffatome und mindestens 1 Stickstoff- und 1 Schwefelatom als Heteroatome, vorzugsweise 1 oder 2 Schwefel-, 1 bis 5 Stickstoff- und gegebenenfalls 1 oder 2 Sauerstoffatome, insbesondere 1 Schwefel-, 2-Stickstoff- und 1 Sauerstoffatom auf. Verbindungsglieder mit einem Kohlenstoffatom sind besonders bevorzugt. Je nach Anzahl der hydrophoben Substituenten enthalten die Leimungsmittel 1 bis 4, vorzugsweise 1 oder 2, insbesondere 1 Verbindungsglied der angegebenen Art.

Bevorzugte Verbindungsglieder entsprechen im allgemeinen einer der Formeln

(1) 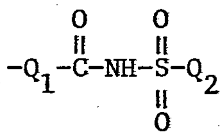 ,

(2) 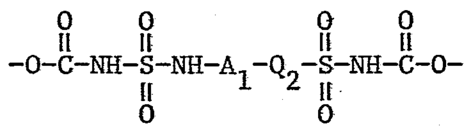 oder

(3) 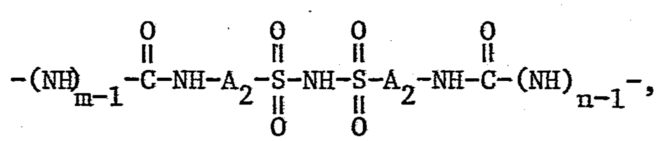 ,

worin $A_1$ Aethylen oder Propylen, $A_2$ unsubstituiertes oder durch Halogen, $C_1$-$C_4$-Alkyl oder -Alkoxy substituiertes Phenyl, $Q_1$ -O-, -NH- oder $-N<$ , $Q_2$ -NH- oder $-N<$ und m und n voneinander verschieden oder vorzugsweise gleich sind, und je 1 oder 2 bedeuten. Das Verbindungsglied der Formel (2) stellt, sofern $Q_1$ -NH- ist, einen zweiwertigen Rest dar. Ist aber $Q_1$ $-N<$, so stellt das Verbindungsglied der Formel (2) hingegen einen dreiwertigen Rest dar.

In den Formeln (1), (2) und (3) stellt die Imingruppe, welche der $-SO_2$-Gruppe benachbart ist bzw. zwischen den beiden $SO_2$-Gruppen steht, die acide Gruppe des Leimungsmittels dar. Die charakteristische Gruppe $-SO_2$-NH-CO- in den Verbindungsgliedern der Formeln (1) und (2) leiten sich z.B. von Sulfonylisocyanaten, insbesondere vom Chlorsulfonylisocyanat ab. In Formel (2) ist der Rest $A_1$ Teil eines aliphatischen Brückengliedes, welcher bevorzugt der Formel

(4) 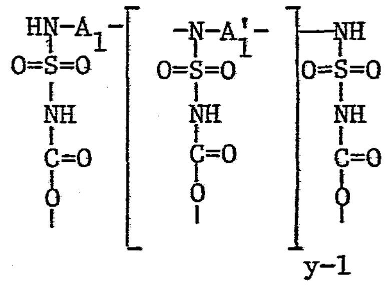

entspricht, worin $A_1$ und $A_1'$ voneinander verschieden oder vorzugsweise gleich sind und je Aethylen oder Propylen und y eine ganze Zahl von 1 bis 5, insbesondere 2, bedeuten.

In Formel (3) ist der Rest $A_2$ Teil eines aromatischen Brückengliedes, welcher der Formel

(5) 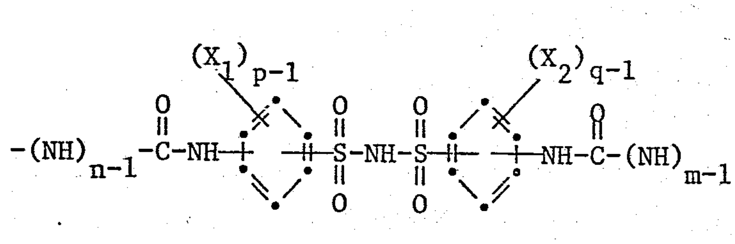

worin $X_1$ und $X_2$ voneinander verschieden oder vorzugsweise gleich sind und je Halogen, vorzugsweise Brom, insbesondere Chlor oder vor allem $C_1$-$C_4$-Alkyl oder -Alkoxy, vorzugsweise Methyl, insbesondere Methoxy und m, n, p und q voneinander verschieden oder vorzugsweise gleich sind und je 1 oder 2 bedeuten.

Von besonderer Bedeutung als Leimungsmittel (A) sind vor allem solche, die durch chemische Umsetzung von mindestens

($a_1$) 1 Mol Chlorsulfonylisocyanat mit

($b_1$) etwa 1 Mol eines Fettalkohols und hierauf mit

($b_2$) etwa 1 Mol eines aromatischen Monoamins, eines primären oder sekundären Fettamins, eines Alkylendiamins oder eines Polyalkylenpolyamins, oder von

($a_1$) 1 Mol Chlorsulfonylisocyanat mit

($b_2$) etwa 2 Mol eines primären oder sekundären Fettamins oder von

($a_2$) 1 Mol eines unsubstituierten oder mit Halogen oder $C_1$-$C_4$-Alkyl oder -Alkoxy substituierten Diamino-diphenyl-disulfimids mit

($b_3$) etwa 2 Mol eines Fettsäurehalogenids und/oder Alkyl- oder -Alkenylisocyanats

erhältlich sind.

Beim Einsatz der Komponente ($b_1$) oder ($b_3$) oder eines Fettamins oder aromatischen Amins als Komponente ($b_2$) sind hingegen monomere Leimungsmittel erhältlich, welche gegenüber den oligomeren Leimungsmitteln auch bevorzugt sind.

Bevorzugte Diamino-diphenyl-disulfimide als Komponente ($a_2$), aus welchen die Leimungsmittel (A) erhältlich sind, sind unsubstituiert

oder vorzugsweise mit Brom oder insbesondere Chlor sowie vor allem mit Methyl oder insbesondere Methoxy substituiert.

Als spezifische Vertreter der Komponente ($a_2$) seien beispielsweise 4,4'-, 3,3'-, 3,4'- und 3,5-Diamino-diphenyl-disulfimid, 3,3'-Diamino-4,4'-dichlor-diphenyl-disulfimid, 4,4'-Diamino-3,3'-dichlor-diphenyl-disulfimid, 3,3'-Diamino-4-chlor-diphenyl-disulfimid, 3,5-Diamino-4-chlor-diphenyl-disulfimid, 3,5-Diamino-diphenyl-4-methyl-disulfimid und insbesondere 3,3'-Diamino-4,4'-dimethoxy-diphenyldisulfimid genannt. Die Komponente ($a_2$) der angegebenen Art ist an sich bekannt und z.B. in der deutschen Offenlegungsschrift 2 000 927 beschrieben. Diese Druckschrift beschreibt auch das Herstellungsverfahren der Komponente ($a_2$).

Als Komponente ($b_1$), aus welchen das Leimungsmittel (A) erhältlich ist, kommen vor allem ungesättigte, vorzugsweise gesättigte aliphatische Alkohole mit 6 bis 22, vorzugsweise 8 bis 22, insbesondere 16 bis 20 Kohlenstoffatomen in Betracht. Sofern die Komponente ($b_2$) ein Fettamin ist, so handelt es sich im allgemeinen um Mono- oder Dialkylamine oder Mono- oder Dialkenylamine mit jeweils 6 bis 22, vorzugsweise 8 bis 22, insbesondere 16 bis 20 Kohlenstoffatomen im Alkyl- oder Alkenylrest. Als spezifische Vertreter von $C_{16}$-$C_{20}$-Fettalkoholen und von Mono- oder Dialkylaminen mit $C_{16}$-$C_{20}$-Alkylresten für die Komponenten ($b_1$) und ($b_2$) seien ihrer guten Zugänglichkeit wegen Hexadecanol, Octadecanol, Oleylalkohol, Octadecylamin und Dioctadecylamin erwähnt. Auch technische Gemische der Fettalkohole zw. der Fettamine der angegebenen Art kommen in Betracht.

Sofern ein aromatisches Monoamin als Komponente ($b_2$) eingesetzt wird, handelt es sich vor allem um solche der Benzolreihe, die gegebenenfalls mit 1, 2 oder 3 Methylgruppen substituiert sind. Als spezifische Vetreter solcher Amine seien Mesidin, 1-Amino-2,6-, -2,3- und -3,4-dimethyl-benzol, m- und p-Xylidin, vor allem Anilin und insbesondere o-, m- und p-Toluidin genannt.

Sofern Alkylendiamine oder Polyalkylenpolyamine als Komponente ($b_2$) eingesetzt werden, entsprechen sie in ihrer bevorzugten Ausführungsform der Formel

(6) $$H_2N-A_1-[NH-A_1']_{y-1}-NH_2,$$

worin $A_1$ und $A_1'$ je Propylen oder vorzugsweise Aethylen und y eine ganze Zahl von 1 bis 5 bedeuten. Als spezifische Vertreter seien z.B. Tetraäthylenpentamin, Triäthylentetramin, vor allem Aethylendiamin und insbesondere Diäthylentriamin erwähnt.

Die Fettalkohole und Fettamine der vorstehend angegebenen Art als als Komponente ($b_1$) und ($b_2$) und insbesondere die Fettsäurehalogenide als Komponente ($b_3$) leiten sich strukturell von ungesättigten oder gesättigten $C_6$-$C_{22}$, vorzugsweise $C_8$-$C_{22}$-, insbesondere $C_{16}$-$C_{20}$-Fettsäuren ab. Bei den Fettsäurehalogeniden handelt es sich z.B. um diejenigen der Capron-, vorzugsweise Capryl-, Caprin-, Laurin-, Myristin- oder Myristolein-, Palmitolein-, Elaeostearin-, Clupanodonsäure, insbesondere Oel-, Elaidin-, Eruka-, Linol- und Linolensäure. Hierbei kommt den Halogeniden der Palmitin-, Stearin-, Oel- und Behensäure eine besondere Bedeutung zu, wobei Palmitin- und vor allem Stearinsäurehalogenide im Vordergrund des Interesses stehen. Auch technische, gut zugängliche Gemische der soeben erwähnten Säurehalogenide kommen in Betracht. Als Fettsäurehalogenide für die Komponente ($b_3$) sind Bromide und vor allem Chloride bevorzugt. Somit stehen als Komponente ($b_3$) Behensäurechlorid, Oelsäurechlorid, vor allem Palmitinsäurechlorid und insbesondere Stearinsäurechlorid im Vordergrund des Interesses.

Ist die Komponente ($b_3$) ein Alkyl- oder Alkenylisocyanat, so leiten sich solche Isocyanate von primären Fettaminen, d.h. von N-Monoalkyl- oder N-Monoalkenylaminen ab. Von Interesse sind vor allem aliphatische Isocyanate mit 6 bis 22, vorzugsweise 8 bis 22, insbesondere 16 bis 20 Kohlenstoffatomen im Alkyl- oder Alkenylrest. Von grösstem Interesse sind vor allem Palmityl- und insbesondere Stearylisocyanat sowie deren technische Gemische.

Bevorzugte Leimungsmittel (A) der angegebenen Art weisen Molekulargewichte von etwa 400 bis etwa 3000, vorzugsweise etwa 600 bis etwa

1500 und infolge ihres Gehaltes an mindestens einer aciden -NH-Gruppe eine Säurezahl (mg KOH/g Substanz) von etwa 15 bis etwa 150, vorzugsweise etwa 50 bis etwa 110 auf.

Die an sich neuen Verbindungen, die als erfindungsgemässe Leimungsmittel (A) verwendbar sind, entsprechen einer der Formeln

(7) 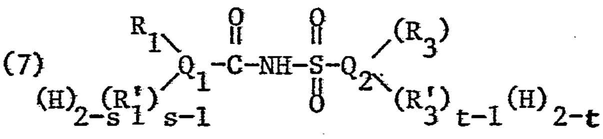 ,

(8) 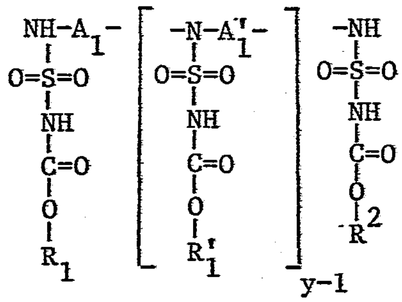 oder

(9) 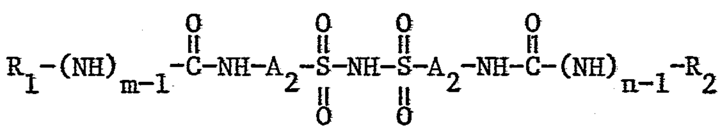 ,

worin $R_1$, $R_1'$ und $R_2$ voneinander verschieden oder vorzugsweise gleich sind und je Alkyl oder Alkenyl mit 6 bis 22, vorzugsweise 8 bis 22, insbesondere 16 bis 20 Kohlenstoffatomen, $R_3$ und $R_3'$ voneinander verschieden oder vorzugsweise gleich sind und die für $R_1$ und $R_2$ angegebenen Bedeutungen haben oder unsubstituiertes oder durch Methyl substituiertes Phenyl, vorzugsweise Xylyl, insbesondere Tolyl und s und t voneinander verschieden oder vorzugsweise gleich sind und je 1 oder 2 bedeuten, und $A_1$, $A_1'$, $A_2$, $Q_1$, $Q_2$, m und n die angegebenen Bedeutungen haben, wobei s und t 2 sind, sofern $Q_1$ und $Q_2$ für $-N<$ stehen.

Bevorzugte Verbindungen entsprechen einer der Formeln

(11) 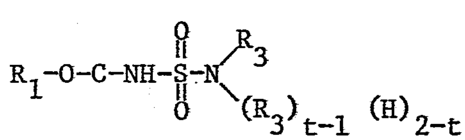 ,

(12) 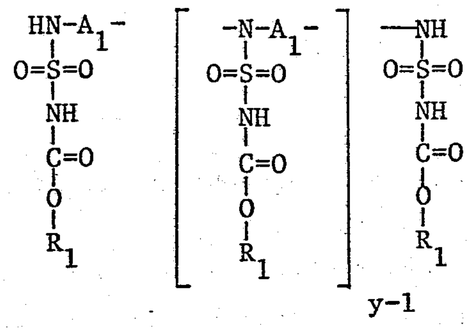 oder

(13) 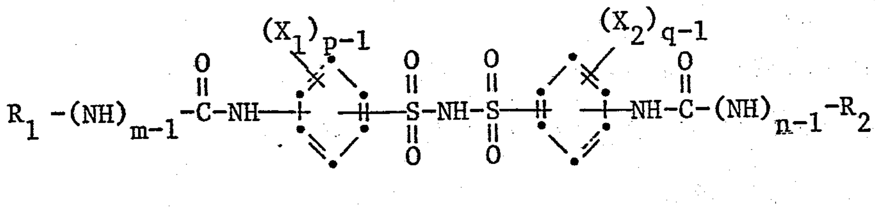 ,

worin $A_1$, $R_1$, $R_2$, $R_3$, $X_1$, $X_2$, m, n, q, p, t und y die angegebenen Bedeutungen haben.

Verbindungen, die im Vordergrund des Interesses stehen, entsprechen den Formeln

(14) 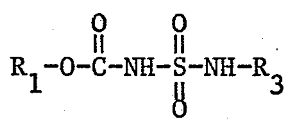 ,

(15) 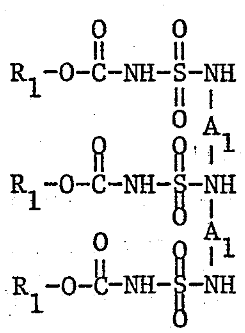 oder

(16) 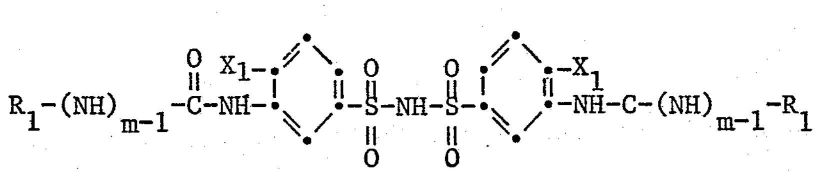 ,

vor allem der Formel

(17) 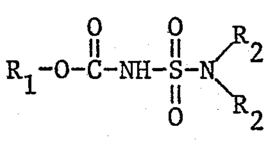

und insbesondere der Formel

(18) 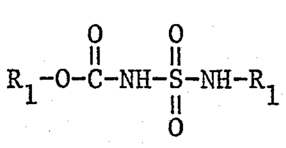 oder

(19) 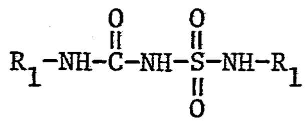

worin $A_1$, $R_1$, $R_2$, $R_3$, X und m die angegebenen Bedeutungen haben.

Das Verfahren zur Herstellung der Verbindungen der Formeln (7), (8) und (9) zeichnet sich dadurch aus, dass man nach an sich bekannten Methoden entweder

($a_1$) 1 Mol Chlorsulfonylisocyanat mit

($b_1$) etwa 1 Mol eines Fettalkohols der Formel

(20) $R_1 - OH$,

worin $R_1$ die angegebenen Bedeutungen hat, und hierauf mit

($b_2$) etwa 1 Mol eines Alkylendiamins oder Polyalkylenpolyamins der Formel (6) oder eines primären oder sekundären Fettamins der Formeln

(21) 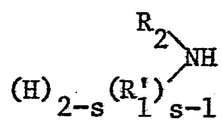 oder

(22) 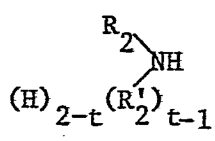 ,

worin s, t, $R_1$, $R_1'$ und $R_2$ die angegebenen Bedeutungen haben, $R_2'$ die für $R_1'$ angegebenen Bedeutungen hat und $R_2$ und $R_2'$ voneinander verschieden oder vorzugsweise gleich sind oder eines aromatischen Monoamins der Formel

(23) $R_4 - NH_2$ ,

worin $R_4$ unsubstituiertes oder durch Methyl substituiertes Phenyl bedeutet, oder

($a_1$) 1 Mol Chlorsulfonylisocyanat mit

($b_2$) etwa 2 Mol eines primären oder sekundären Fettamins der Formel (21), oder

($a_2$) 1 Mol eines Diamino-diphenyl-disulfimides der Formel

(24) 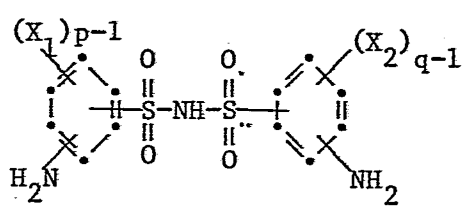 ,

worin $X_1$, $X_2$, p und q die angegebenen Bedeutungen haben, mit
($b_3$) etwa 2 Mol eines Fettsäurehalogenides der Formel

(25) $R_1-\overset{O}{\overset{\|}{C}}-Z_1$ oder

(26) $R_2-\overset{O}{\overset{\|}{C}}-Z_2$

oder eines Alkyl- oder Alkenylisocyanates der Formel

(27) $R_1-N=C=O$ oder

(28) $R_2-N=C=O$ oder deren Gemische,

worin $Z_1$ und $Z_2$ voneinander verschieden oder vorzugsweise gleich sind und je Halogen, vorzugsweise Brom, insbesondere Chlor bedeuten und $R_1$ und $R_2$ die angegebenen Bedeutungen haben, miteinander umsetzt.

Die Umsetzungen der Komponente ($a_1$) mit den Komponenten ($b_1$) und ($b_2$) und der Komponente ($a_2$) mit der Komponente ($b_3$) werden vorzugsweise bei Temperaturen von höchstens 90°C, vorzugsweise -10 bis +60°C, insbesondere 40 bis 60°C und im allgemeinen in Gegenwart eines Lösungsmittels, das gegenüber jedem der Ausgangs-, Zwischen- und Endprodukte inert sein muss, durchgeführt. Als Beispiele möglicher Lösungsmittel seien Aether, wie Diäthyläther, Diisopropyläther oder gegebenenfalls halogenierte Kohlenwasserstoffe wie etwa Dichloräthan, Tetrachlorkohlenstoff, ferner Benzol, Toluol, Chlorbenzol, o-, m- und p-Xylol, ein technisches Xylolgemisch oder auch Gemische der erwähnten Kohlenwasserstoffe genannt.

Beim Umsetzen der aus den Komponenten ($a_1$) und ($b_1$) erhaltenen Zwischenprodukte mit der Komponente ($b_2$) oder beim Umsetzen der Komponente ($a_2$) mit der Komponente ($b_3$), sofern diese ein Säurehalogenid ist, empfiehlt es sich, zur Verhinderung der Bildung von Nebenprodukten eine mindestens äquimolare Menge (bezogen auf das primäre Fettamin), vorzugsweise jedoch einen Ueberschuss an einer schwachen, stickstoffhaltigen Base, z.B. Pyridin, Triäthylamin, Isochinolin oder Chinolin mitzuverwenden. Pyridin weist den Vorteil auf, als Reaktionsmedium an Stelle des Lösungsmittels der vorstehend angegebenen Art eingesetzt werden zu können, worin die Ausgangskomponenten suspendiert werden können.

Sofern $R_1$, $R_1'$, $R_2$ und $R_2'$ in den Formeln (20), (21), (22) und (25) bis (28) für einen Alkenylrest der angegebenen Art stehen, ist es zudem vorteilhaft, die Umsetzung in inerter Stickstoffatmosphäre durchzuführen, wobei bei höheren Temperaturen von z.B. über 90°C die Gegenwart eines Polymerisationsinhibitors, z.B. Methylenblau, Benzthiazin oder vor allem Hydrochinon, auch vorteilhaft sein kann.

Sofern die Komponente ($b_1$) eingesetzt wird, werden in der Regel die Umsetzungen zweistufig durchgeführt, wobei in einer ersten Stufe der Fettalkohol der Formel (2) mit Chlorsulfonylisocyanat umgesetzt wird, und der so erhaltene Ester in einer zweiten Stufe anschliessend mit einem primären oder sekundären Fettamin der Formel (21) oder (22), einem aromatischen Monoamin der Formel (23) oder einem Alkylendiamin oder Polyalkylendiamin der Formel (6) weiter umgesetzt wird.

Vor ihrem Einsatz als Komponente (A) in erfindungsgemässen Papierleimungsverfahren brauchen die Leimungsmittel nach erfolgter Herstellung im allgemeinen nicht gereinigt oder umkristallisiert zu werden, sondern können in der Regel direkt verwendet werden.

Vor allem bei separater Zugabe (in beliebiger Reihenfolge) des Leimungsmittels (A) und des Retentionsmittels (B) zur Faserstoffsuspension beim erfindungsgemässen Verfahren zum Leimen von Papier oder Karton ist es zweckmässig, das Leimungsmittel teilweise in Salzform einzusetzen. Solche Salze können bei Bedarf dadurch erhalten werden, dass man nach beendeter Umsetzung der Komponenten ($a_1$), ($b_1$) und ($b_2$) oder ($a_1$) und ($b_2$) oder ($a_2$) und ($b_3$) die erhaltenen Umsetzungsprodukte durch Zugabe u.a. eines Alkylamins oder Alkanolamins mit insgesamt höchstens 6 Kohlenstoffatomen, z.B. Trimethylamin, Triäthylamin, Monoethanolamin, Diäthanolamin, vor allem durch Zugabe von Ammoniak oder eines Alkalimetallhydroxydes, beispielsweise Kalium- oder vor allem Natriumhydroxyd, in der Regel in wässrigem Medium bei Raumtemperatur (etwa 15 bis etwa 25°C) in die entsprechenden Salze gegebenenfalls mindestens teilweise überführt. Zweckmässigerweise wird ein Alkalimetallhydroxyd, z.B. Kalium- oder vor allem Natriumhydroxyd oder insbesondere Ammoniak in der Regel in Form ihrer verdünnten, etwa 1 bis 10 gewichtsprozentigen,

wässrigen Lösungen verwendet. Zweckmässig wird in der Regel höchstens 2 Mol, vor allem höchstens 1 Mol, vorzugsweise 0,1 bis 0,9, insbesondere 0,2 bis 0,7 Mol Ammoniak oder Alkalihydroxyd pro vorhandene acide Imingruppe des Leimungsmittels eingesetzt. Die als Salze vorliegenden Leimungsmittel weisen somit acide $-\overset{|}{N}N$ Gruppen auf, die mindestens teilweise in die Gruppe $-\underline{\overset{|}{N}}^{\ominus}M^{\oplus}$ überführt werden, worin $M^{\oplus}$ die entsprechenden Amin-, Ammonium- oder Alkalimetall-Kationen bedeutet.

Im erfindungsgemässen Papierleimungsverfahren wird neben dem an sich neuen, vorstehend beschriebenen monomeren bis oligomeren, anionischen oder aciden Leimungsmittel (A) stets ein polymeres, kationisches Retentionsmittel (B) eingesetzt, welches in der Regel ein Molekulargewicht von mindestens etwa 1000, vorzugsweise etwa 2000 bis etwa 2'000'000 aufweist. Retentionsmittel mit Molekulargewichten im Bereich von 10'000 bis 100'000 sind besonders bevorzugt. Grundsätzlich kommt jedes handelsübliche Retentionsmittel in Betracht für seinen Einsatz im erfindungsgemässen Verfahren. Als Beispiele herkömmlicher Retentionsmittel (B), die sich besonders gut dazu eignen, zusammen mit dem Leimungsmittel (A) im erfindungsgemässen Papierleimungsverfahren eingesetzt zu werden, seien Polyalkylenimine, Epihalogenhydrin-Addukte von Umsetzungsprodukten aus Polyalkylenpolyaminen und aus aliphatischen Dicarbonsäuren oder von Umsetzungsprodukten aus Polyalkylenpolyaminen, aus Dicyandiamid und gegebenenfalls aus unveresterten oder mit Alkanolen veresterten, organischen Dicarbonsäuren, Umsetzungsprodukte aus Dicyandiamid, aus Formaldehyd, aus Ammoniumsalzen starker anorganischer Säuren und aus Alkylendiaminen oder Polyalkylenpolyaminen, kationisch modifizierte Stärken oder Kohlenhydrate aus Johannisbrot- oder Guarkernmehl, Copolymerisate auf Basis von Polyamid-Aminen und Umsetzungsprodukte aus Epihalogenhydrinen und aus polymerisierten Diallylaminen erwähnt.

Bevorzugte Epichlorhydrin-Addukte von Umsetzungsprodukten aus Polyalkylenpolyaminen und aliphatischen Dicarbonsäuren sind z.B. in der britischen Patentschrift 865 727, Epichlorhydrin-Addukte aus Umsetzungsprodukten aus Dicyandiamid und Diäthylentriamin oder Triäthylentetramin

z.B. in der deutschen Offenlegungsschrift 2 710 061 und in der britischen Patentschrift 1 125 486, Epichlorhydrin-Addukte von Umsetzungsprodukten aus Diäthylentriamin, Dicyandiamid und unveresterten oder vorzugsweise mit Niederalkanolen veresterten Dicarbonsäuren, insbesondere Dimethyladipat, z.B. in der britischen Patentschrift 1 125 486 und Umsetzungsprodukte aus Dicyandiamid, Formaldehyd, Ammoniumsalzen starker anorganischer Säuren und aus Aethylendiamin oder Triäthylentetramin, z.B. in der US Patentschrift 3 491 064 beschrieben. Bevorzugte kationisch modifizierte Stärken oder Kohlenhydrate aus Johannisbrot- oder Guarkernmehl sind Alkylenoxyd-Addukte dieser Stärken oder Kohlenhydrate, wobei das eingesetzte Alkylenoxyd 2 oder 3 Kohlenstoffatome im Alkylenrest und quaternäre Ammoniumgruppen aufweist. Copolymerisate auf Basis von Polyamid-Aminen weisen Molekulargewichte von $10^3$ bis $10^5$, vorzugsweise $10^3$ bis $10^4$ auf und sind z.B. aus aliphatischen, gesättigten Dicarbonsäuren mit 2 bis 10, vorzugsweise 3 bis 6 Kohlenstoffatomen, insbesondere Adipinsäure, und Polyalkylenpolyaminen, z.B. Polypropylen- und Polyäthylenpolyamin, insbesondere Dimethylaminohydroxypropyl-diäthylentriamin, erhältlich. Sie sind z.B. in CTFA Cosmetic Ingredient Dictionary, 3. Auflage 1982, der Cosmetic Toiletry and Fragrance Association beschrieben. Umsetzungsprodukte aus Epihalogenhydrinen und polymerisierten Diallylaminen weisen bevorzugt Molekulargewichte von 1000 bis 2000 auf und sind z.B. in den US-Patentschriften 3 700 623 und 4 279 794 beschrieben.

Als Retentionsmittel (B), die zur Verwendung zusammen mit den Leimungsmitteln (A) im erfindungsgemässen Papierleimungsverfahren im Vordergrund des Interesses stehen, sei eine mit einem quaternäre Ammoniumgruppen enthaltenden Propylenoxyd modifizierte Mais- oder Kartoffelstärke, deren 25 %ige Anschlämmung in destilliertem Wasser bei 20°C einen pH-Wert von 4,2 bis 4,6 aufweist, ein Polyäthylenimin, das ein Molekulargewicht von 10 000 bis 100 000 aufweist, ein Epichlorhydrin-Addukt eines Umsetzungsproduktes aus Triäthylentetramin und Dicyandiamid, ein Epichlorhydrin-Addukt eines Umsetzungsproduktes aus Diäthylentriamin, Dicyandiamid und Dimethyladipat, ein Umsetzungsprodukt aus Dicyandiamid, Formaldehyd, Ammoniumchlorid und Aethylendiamin, ein Epichlorhydrin-Addukt eines Poly-N-Methyldiallylamins und ein Copolymerisat aus Adipinsäure und Dimethylamino-hydroxypropyl-diäthylentriamin genannt.

Verfahrensgemäss werden in der Regel 0,02 bis 3, vorzugsweise 0,1 bis 3, insbesondere 0,2 bis 0,8 Gewichtsprozent des Leimungsmittels (A) und 0,02 bis 3, vorzugsweise 0,1 bis 3, insbesondere 0,2 bis 0,4 Gewichtsprozent des Retentionsmittels (B), bezogen jeweils auf Trokkensubstanz an (A) und (B) und auf den Feststoffgehalt der Faserstoffsuspension, eingesetzt. 0,02 bis weniger als 0,1 Gewichtsprozent des Leimungsmittels (A) und des Retentionsmittels (B) reichen nur für das sogenannte "size press control", das mit konventionellen Leimungstests nicht erfassbar ist (vgl. z.B. Artikel "Control and understanding of size press pickup" von D.R. Dill in der Zeitschrift TAPPI (Proceedings of the Technical Association of the Pulp and Paper Industry), Band 57, Nr. 1 von Januar 1974, Seiten 97 bis 100).
Die Faserstoffsuspension, zu welcher die Leimungsmittel (A) und die Retentionsmittel (B) gegeben werden, weist in der Regel einen Feststoffgehalt von 0,1 bis 5, vorzugsweise 0,3 bis 3, insbesondere 0,3 bis 1 Gewichtsprozent und einen Schopper-Riegler-Mahlgrad von etwa 10° bis etwa 60°, vor allem 20 bis 60°, vorzugsweise 20 bis 45°, insbesondere 25 bis 35° auf. Sie enthält in der Regel Zellstoff, insbesondere solchen aus Nadelholz, z.B. Kiefernholz, oder aus Hartholz, d.h. Laubholz z.B. Buchenholz, der nach herkömmlichen Verfahren, z.B. dem Sulfit- oder vor allem dem Sulfatverfahren hergestellt wird. Zudem enthält die Faserstoffsuspension gegebenenfalls Holzschliff. Auch alaunhaltiges Altpapier kann in der Faserstoffsuspension enthalten sein. Auch Zellstoffsuspensionen, die nach dem sogenannten CMP- oder CTMP-Verfahren (Chemimechanical und chemithermomechanical pulping processes, vgl. z.B. Artikel "Developments in refiner mechanical

pulping" von S.A. Collicutt und Mitarbeitern in TAPPI, Band 64, Nr. 6 von Juni 1981, Seiten 57 bis 61) hergestellt werden, kommen in Betracht.

Die Faserstoffsuspension kann zudem organische oder mineralische Füllmittel enthalten. Als organische Füllmittel kommen u.a. synthetische Pigmente, z.B. Polykondensationsprodukte aus Harnstoff oder Melamin und Formaldehyd mit grossen spezifischen Oberflächen, die in hochdisperser Form vorliegen und u.a. in den britischen Patentschriften 1 043 937 und 1 318 244 beschrieben sind, als mineralische Füllmittel u.a. Montmorillonit, Titandioxyd, Calciumsulfat und vor allem Talk, Kaolin und/oder Kreide (Calciumcarbonat) in Betracht. In der Regel enthält die Faserstoffsuspension 0 bis 40, vorzugsweise 5 bis 25, insbesondere 15 bis 20 Gewichtsprozent, bezogen auf den Feststoffgehalt der Faserstoffsuspension, an Trokkensubstanz der Füllmittel der angegebenen Art.

Der pH-Wert der Faserstoffsuspension kann in einem weiten Bereich liegen, wobei z.B. Werte von 3,5 bis etwa 10 vorliegen können.

Bei Zusatz von z.B. Calciumcarbonat werden alkalische Faserstoffsuspensionen mit einem pH-Wert von etwa 7 bis etwa 9, vorzugsweise 7,5 bis 8,5 erhalten. Saure Faserstoffsuspensionen mit einem pH-Wert von 3,5 bis 7, vorzugsweise 5 bis 7, insbesondere 5 bis 6, können in Abwesenheit von Calciumcarbonat durch Zugabe von Säuren, z.B. Schwefel- oder Ameisensäure oder vor allem von z.B. latent sauren Sulfaten, wie Aluminiumsulfat (Alaun), erhalten werden.

Faserstoffsuspensionen, die kein Füllmittel enthalten, können in einem breiten pH-Bereich von z.B. 3,5 bis 10 vorliegen. Bevorzugt sind Faserstoffsuspensionen, die gegebenenfalls durch Zusatz von Kreide einen pH-Wert von etwa 7 bis etwa 9 aufweisen und insofern vorteilhaft sind, dass mögliche Korrosionserscheinungen an den empfindlichen Papiermaschinen ausgeschlossen werden.

Die Faserstoffsuspension kann auch Additive enthalten, wie z.B. Stärke oder ihre Abbauprodukte, welche die Faser/Faser- oder Faser/Füllmittel-Bindung erhöhen.

Auch hochmolekulare Polymere der Acrylsäurereihe, z.B. Polyacrylamide, mit Molekulargewichten über 1'000'000 können zu den Faserstoffsuspensionen als Hilfsmittel zum Zurückhalten feinster Zellstoffaserteilchen gegeben werden, wobei minimale Einsatzmengen von etwa 0,005 bis 0,02 Gewichtsprozent, bezogen auf Trockensubstanz des Polymeres und den Feststoffgehalt der Faserstoffsuspensionen, genügend sind.

Die Faserstoffsuspension wird im erfindungsgemässen Verfahren auf an sich bekannte Weise auf Blattbildnern oder vorzugsweise kontinuierlich auf Papiermaschinen üblicher Bauart zu Papier oder Karton weiterverarbeitet. Nach einer Trocknung bei etwa 100 bis 140°C während etwa 0,5 bis 10 Minuten werden Papiere eines variablen Flächengewichtes von z.B. 50 bis 200 $g/m^2$ erhalten.

Wie eingangs erwähnt, enthält die wässrige Zusammensetzung zur Durchführung des erfindungsgemässen Papierleimungsverfahrens neben fakultativen üblichen Zusätzen das Leimungsmittel (A), sofern das Leimungsmittel und das Retentionsmittel (B) separat zur Faserstoffsuspension gegeben werden. In diesem Fall enthält die Zubereitung das Leimungsmittel in der Regel teilweise in Form seiner Salze (erhalten unter Mitverwendung von z.B. Ammoniak, eines Alkyl- oder Alkanolamins oder eines Alkalimetallhydroxydes der angegebenen Art in den vorstehend angegebenen Verhältnissen). Im allgemeinen enthalten solche Zusammensetzungen 5 bis 30, vorzugsweise 5 bis 20 Gewichtsprozent an Trockensubstanz des teilweise in Salzform vorliegenden Leimungsmittels, bezogen auf das Gewicht der wässrigen Zusammensetzung.

Hingegen enthält die wässrige Zusammensetzung neben den fakultativen, üblichen Zusätzen, sofern das Leimungsmittel (A) und das Retentionsmittel (B) gleichzeitig zur Faserstoffsuspension gegeben werden,

(A) 2 bis 40, vorzugsweise 5 bis 30, insbesondere 5 bis 10 Gewichtsprozent Leimungsmittel (berechnet als Trockensubstanz), bezogen auf das Gewicht der wässrigen Zusammensetzung, wobei das Leimungsmittel gegebenenfalls in Salzform vorliegt, und

(B) 0,1 bis 20, vorzugsweise 0,5 bis 10, insbesondere 3 bis 8 Gewichtsprozent Retentionsmittel (berechnet als Trockensubstanz), bezogen auf die wässrige Zusammensetzung.

Die wässrigen Zusammensetzungen der angegebenen Art enthalten gegebenenfalls als übliche Zusätze oberflächenaktive Verbindungen, z.B. Dispergatoren oder ferner Emulgatoren und/oder wasserlösliche, organische Lösungsmittel. Als Dispergatoren und Emulgatoren kommen z.B. herkömmliche Ligninsulfonate, Aethylenoxydaddukte von Alkylphenolen, Fettaminen, Fettalkoholen oder Fettsäuren, Fettsäureester mehrwertiger Alkohole, substituierte Benzimidazole oder Kondensationsprodukte aus aromatischen Sulfonsäuren und Formaldehyd in Betracht. Weitere oberflächenaktive Verbindungen sind vorzugsweise die anionischen Tenside, insbesondere Sulfattenside, z.B. Diäthanolaminlaurylsulfat oder äthoxylierte Laurylsulfate. Mögliche wasserlösliche, organische Lösungsmittel sind aliphatische Aether mit 1 bis 10 Kohlenstoffatomen, z.B. Dioxan, Methylenglykol-n-butyläther oder Diäthylenglykolmonobutyläther oder Alkohole mit 1 bis 4 Kohlenstoffatomen, z.B. Isopropanol, Aethanol oder Methanol.

Die Zusammensetzungen werden auf übliche Weise hergestellt, indem man das Leimungsmittel (A) zusammen mit dem Retentionsmittel (B) oder das Leimungsmittel (A) in der Regel teilweise in Form seines

Salzes für sich allein entweder in geschmolzenem Zustand oder vorzugsweise in festem Zustand, insbesondere in pulverisierter Form, in der Regel in Gegenwart von Glasperlen und nötigenfalls von Emulgatoren (bei Leimungsmittel in geschmolzenem Zustand) oder Dispergatoren (bei Leimungsmittel in Pulverform) bei höchstens 90°C, vorzugsweise etwa 50 bis etwa 85°C bei Emulsionen, insbesondere bei etwa 15 bis etwa 25°C bei Dispersionen, verrührt, wobei lagerstabile, homogene, weiterverdünnbare Emulsionen oder vorzugsweise Dispersionen erhalten werden. Da die Leimungsmittel zusammen mit den Retentionsmitteln oder die teilweise als Salze vorliegenden Leimungsmittel in der Regel selbst-dispergierend oder selbst-emulgierend sind, ist der Einsatz von Dispergatoren oder Emulgatoren im allgemeinen nicht unbedingt erforderlich. Dies gilt auch für den fakultativen Zusatz von Lösungsmitteln und/oder Tensiden, die nur bei ungenügender Lagerstabilität der Dispersionen oder Emulsionen eingesetzt werden.

Als Vorteil des erfindungsgemässen Verfahrens sei erwähnt, dass Faserstoffsuspensionen der verschiedensten Art mit relativ kleinen Mengen an Leimungs- und Retentionsmittel auf einfache Art und Weise zu Papier verarbeitet werden können, welches gute Leimungseigenschaften (Tintenschwimmdauer und vor allem Wasseraufnahme nach Cobb) aufweist. Das verfahrensgemäss geleimte Papier weist gute mechanische Eigenschaften d.h. gute Festigkeiten, insbesondere eine gute Reissfestigkeit auf. Eine gute Reproduzierbarkeit des Verfahrens ist gewährleistet. Insbesondere können holzschliffhaltige oder altpapierhaltige Faserstoffsuspensionen verarbeitet werden. Auch die Kompatibilität des erfindungsgemäss verwendeten Leimungsmittels mit den verschiedensten Füllmitteln und auch deren Zusätze, wie z.B. Kaolin oder Alaunen in saurem Bereich der Faserstoffsuspensionen, ist vorteilhaft. Die Leimungsmittel weisen ebenfalls eine vorteilhafte Kompatibilität mit optischen Aufhellern auf. Zudem wird der Weissgrad des geleimten Papiers durch die Leimung nicht wesentlich beeinflusst und kann sogar u.U. verbessert werden. Vor allem ist die in der Regel überraschend hohe Lagerstabilität der Leimungsmitteldispersionen der angegebenen

Art von grossem Vorteil.

Die in den nachfolgenden Ausführungsbeispielen angegebenen Teile und Prozente beziehen sich auf das Gewicht.

Herstellung neuer Verbindungen als Leimungsmittel

Beispiel 1: 42,6 Teile Chlorsulfonylisocyanat (0,3 Mol) werden in 100 Teilen Toluol gelöst. Zu dieser Lösung wird in einer ersten Stufe eine Lösung von 81,3 Teilen Octadecanol (0,3 Mol) in 500 Teilen Toluol innerhalb von 30 Minuten gegeben, wobei die Temperatur des Reaktionsgemisches von selbst auf etwa 45°C steigt. Das Reaktionsgemisch wird nach beendeter Octadecanolzugabe während einer Stunde unter Rühren gehalten, wobei die Temperatur des Reaktionsgemisches auf etwa 25°C fällt. Nun wird anschliessend zum Reaktionsgemisch in einer zweiten Stufe eine Lösung von 81,0 Teilen Octadecylamin (0,3 Mol) und 45,6 Teile Triäthylamin (0,45 Mol oder 50% Ueberschuss bezogen auf Octadecylamin) in 500 Teilen Toluol innerhalb von 30 Minuten gegeben, wobei die Temperatur des Reaktionsgemisches von selbst auf etwa 50°C steigt. Anschliessend wird das Reaktionsgemisch während 5 Stunden bei 50°C unter Rühren gehalten. Das Toluol wird dann unter vermindertem Druck aus dem Reaktionsgemisch abdestilliert. Zur Aufarbeitung wird der Destillationsrückstand mit 1000 Teilen einer wässrigen, 1 N (1-normalen) Salzsäurelösung versetzt und die erhaltene Suspension während 1 Stunde bei 20°C unter Rühren gehalten. Nach dem Filtrieren der Suspension wird das Rohprodukt aus Chloroform umkristallisiert.

Man erhält als farbloses Pulver 135 Teile des Umsetzungsproduktes der Formel

(29) $$CH_3-(CH_2)_{17}-O-\overset{O}{\overset{\|}{C}}-NH-\overset{O}{\overset{\|}{\underset{O}{\underset{\|}{S}}}}-NH-(CH_2)_{17}-CH_3 .$$

Schmelzpunkt: 98-104°C, Säurezahl: 93.

<u>Beispiel 2</u>: Man verfährt wie im Beispiel 1 angegeben, setzt jedoch 72,6 Teile Hexadecanol (0,3 Mol) in der ersten Stufe und 72,3 Teile Hexadecylamin in der zweiten Stufe (anstelle von 81,3 Teilen Octadecanol und 81,0 Teilen Octadecylamin) ein. Man erhält als farbloses Pulver 109 Teile des Umsetzungsproduktes der Formel

(30) $$CH_3-(CH_2)_{15}-O-\overset{O}{\overset{\|}{C}}-NH-\overset{O}{\overset{\|}{\underset{O}{\underset{\|}{S}}}}-CH-(CH_2)_{15}-CH_3 \quad .$$

Schmelzpunkt: 95-102°C, Säurezahl:101.

<u>Beispiel 3</u>: Man verfährt wie im Beispiel 1 angegeben, setzt jedoch 81,0 Teile Octadecylamin (0,3 Mol) (anstelle von 81,3 Teilen Octadecanol) in der ersten Stufe ein. Man erhält als farbloses Pulver 167,4 Teile des Umsetzungsproduktes der Formel

(31) $$CH_3-(CH_2)_{17}-NH-\overset{O}{\overset{\|}{C}}-NH-\overset{O}{\overset{\|}{\underset{O}{\underset{\|}{S}}}}-NH-(CH_2)_{17}-CH_3$$

Schmelzpunkt 95-98°C, Säurezahl: 87

<u>Beispiel 4</u>: Man verfährt wie im Beispiel 1 angegeben, setzt jedoch 156,6 Teile Dioctadecylamin (0,3 Mol) (anstelle von 81,0 Teilen Octadecylamin) in der zweiten Stufe ein und kristallisiert das Rohprodukt aus Aceton um. Man erhält als farbloses Pulver 219,0 Teile des Umsetzungsproduktes der Formel

(32) 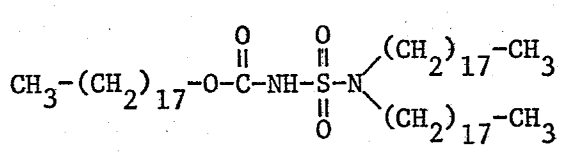

Schmelzpunkt: 58-61°C, Säurezahl: 70.

<u>Beispiel 5</u>: Man verfährt wie im Beispiel 1 angegeben, setzt jedoch 80,5 Teile Oleylalkohol (0,3 Mol) in der ersten Stufe und 32,1 Teile p-Toluidin in der zweiten Stufe ein und kristallisiert das Rohprodukt aus n-Hexan um. Man erhält als ockerfarbene cremige Substanz 87,6 Teile des Umsetzungsproduktes der Formel

(33) 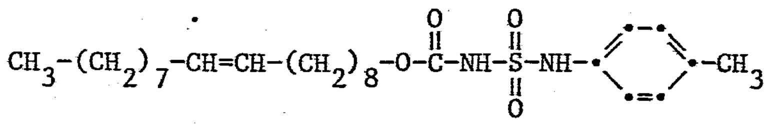 .

Säurezahl: 116.

<u>Beispiel 6</u>: Man verfährt wie im Beispiel 1 angegeben, setzt jedoch 80,5 Teile Oleylalkohol (0,3 Mol) in der ersten Stufe ein und kristallisiert das Rohprodukt aus Aethanol um. Man erhält als gelbliches Pulver 125,4 Teile des Umsetzungsproduktes der Formel

(34) $$CH_2-(CH_2)_7-CH=CH-(CH_2)_8-O-\overset{O}{\overset{\|}{C}}-NH-\underset{\underset{O}{\|}}{\overset{\overset{O}{\|}}{S}}-NH-(CH_2)_{17}-CH_3$$

Schmelzpunkt: 84-89°C, Säurezahl: 87.

<u>Beispiel 7</u>: Man verfährt wie im Beispiel 1 angegeben, setzt jedoch 32,1 Teile p-Toluidin (0,3 Mol) in der zweiten Stufe ein und kristallisiert das Rohprodukt aus Aceton um. Man erhält als gelbliches Pulver 132,3 Teile des Umsetzungsproduktes der Formel

(35) 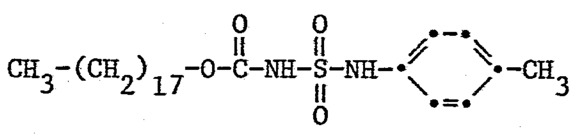 .

Schmelzpunkt: 97-100°C, Säurezahl: 113.

<u>Beispiel 8</u>: Man verfährt wie im Beispiel 1 angegeben, setzt jedoch 10,2 Teile Diäthylentriamin (0,1 Mol) in der zweiten Stufe ein und kristallisiert das Rohprodukt aus Aethanol um. Man erhält als ockerfarbenes Pulver 102 Teile des Umsetzungsproduktes der Formel

(36) 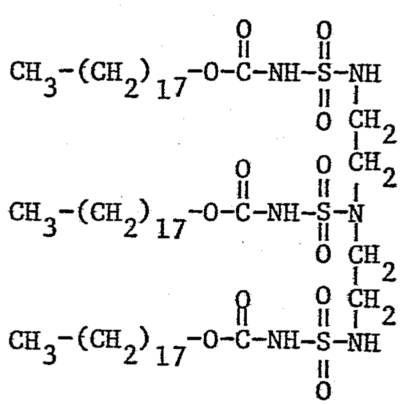 .

Schmelzpunkt: 61-70°C, Säurezahl: 140.

Beispiel 9: 193,7 Teile 3,3'-Amino-4,4'-dimethoxy-diphenyl-disulfimid (0,5 Mol) werden in 2300 Teilen Pyridin suspendiert. Zu dieser Suspension werden 303 Teile Stearinsäurechlorid (1 Mol) gegeben. Das Reaktionsgemisch wird auf 60°C aufgeheizt und 2 Stunden bei dieser Temperatur gehalten, wobei eine Lösung entsteht. Das Rohprodukt fällt beim Kühlen der Reaktionslösung aus und wird abfiltriert. Zur Aufarbeitung wird das Rohprodukt in 3000 Teilen Wasser suspendiert und diese Suspension wird mit 1000 Teilen einer wässrigen, 2-N (2-normalen) Salzsäurelösung versetzt. Das Produkt wird wiederum abfiltriert, mit Aceton nachgewaschen und unter vermindertem Druck bei 60° bis 70°C getrocknet. Man erhält als beigefarbenes Pulver 417 Teile des Umsetzungsproduktes der Formel

(37)

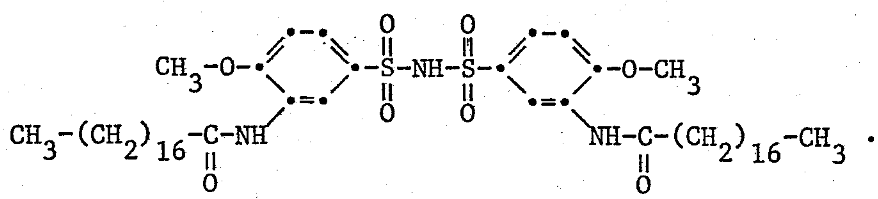

Schmelzpunkt: 148-152°C, Säurezahl: 61

Beispiel 10: Man verfährt wie im Beispiel 9 angegeben, setzt jedoch 296 Teile Stearylisocyanat (anstelle von 303 Teilen Stearinsäurechlorid) ein und führt die Reaktion bei 85°C während 3 Stunden durch. Man erhält als beige gefärbtes Pulver 455 Teile des Umsetzungsproduktes der Formel

(37)

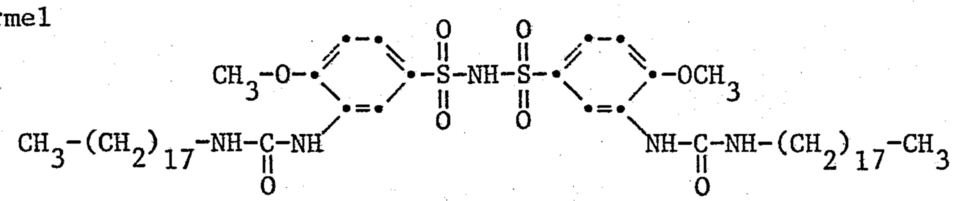

Schmelzpunkt: 180-183°C , Säurezahl: 57

Applikationsbeispiele

Beispiel 11 bis 20: Eine Faserstoffsuspension aus gebleichtem Birkensulfatzellstoff und Kiefernsulfatzellstoff im Gewichtsverhältnis 1:1 in Wasser von 10° dH (deutsche Härtegrade), die einen Schopper-Riegler

Mahlgrad von 35° und einen Feststoffgehalt von 0,5% aufweist, wird mit 20% Kreide als Füllmittel und hierauf mit 0,01% PERCOL 292® (kationenaktives, hochmolekulares (MG >1·$10^7$) Polyacrylamid) als Hilfsmittel zum Zurückhalten feinster Zellstoffaserteilchen versetzt, wobei sich der in der nachfolgenden Tabelle I angegebene pH-Wert der Faserstoffsuspension einstellt. Die Prozentangaben beziehen sich auf Trockensubstanz an Hilfs- und Füllmittel, bezogen auf den Feststoffgehalt der Faserstoffsuspension.

Formulierungen des Leimungsmittels werden hergestellt, indem jeweils 7% der angegebenen Leimungsmittel in Pulverform oder auch als Reaktionsgemische wie sie bei der Herstellung anfallen mit jeweils 3,5% POLYMIN P® (Polyäthylenimin eines Molekulargewichts von 10'000 bis 100'000) als Retentionsmittel in Gegenwart von entionisiertem Wasser und von Glasperlen mit einem Durchmesser von 2 mm bei Raumtemperatur (15 bis 25°C) verrührt werden. Die erhaltenen Dispersionen sind giessbar, homogen und lagerstabil. Die Prozentangaben beziehen sich auf die Trockensubstanz an Leimungs- und Retentionsmittel, bezogen auf das Gesamtgewicht der Formulierung.

Nun wird die wässrige Formulierung des Leimungsmittels und des Retentionsmittels zur Faserstoffsuspension so gegeben, dass die in der nachfolgenden Tabelle I angegebenen Einsatzmengen von 0,5% oder 1% an Trockensubstanz des Leimungsmittels, bezogen auf den Feststoffgehalt der Faserstoffsuspension, entsteht. Anschliessend wird die Faserstoffsuspension in einem Labor-Blattbildner "Formette Dynamique" der Fa. Allimand, Grenoble, Frankreich, zu Papierblättern verarbeitet, die nach der Trocknung bei 130°C während 3 Minuten ein Flächengewicht von 80 g/$m^2$ aufweisen.

Beide Oberflächen der erhaltenen Papierblätter, d.h. die auf der Siebseite des Blattbildners erhaltene Oberfläche und die Gegen- oder Oberseite werden auf ihre Leimungseigenschaften geprüft. Zu diesem Zweck wird die Wasseraufnahme nach Cobb bei 30 Sekunden Einwirkungsdauer (WA $Cobb_{30}$) gemäss DIN 53 132 gemessen. Die Ergebnisse der WA $Cobb_{30}$-Messungen in $g/m^2$ der Siebseite (SS) und Oberseite (OS) nach der Trocknung bei 130°C und nach einer Lagerung von einem Tag bei 20°C sind in der nachfolgenden Tabelle I angegeben. Je geringer die Wasseraufnahme, desto besser ist die Leimung des Papiers. WA $Cobb_{30}$ Werte über 100 entsprechen einer völlig unbefriedigenden Leimung des Papiers.

Tabelle I

| Bei-spiel Nr. | Leimungsmittel | Einsatz-menge (%) | pH-Wert der Suspension | WA $Cobb_{30}$ ($g/m^2$) | | | |
|---|---|---|---|---|---|---|---|
| | | | | nach Trocknung | | nach 1 Tag Lagerung | |
| | | | | SS | OS | SS | OS |
| 11 | Umsetzungsprodukt gemäss Beispiel 1 | 0,5 | 8,8 | 16 | 11 | 14 | 12 |
| 12 | Umsetzungsprodukt gemäss Beispiel 1 | 0,25 | 8,0 | 20 | 15 | 24 | 15 |
| 13 | Umsetzungsprodukt gemäss Beispiel 2 | 0,5 | 9,1 | 17 | 12 | 15 | 11 |
| 14 | Umsetzungsprodukt gemäss Beispiel 3 | 0,5 | 8,8 | 23 | 14 | 20 | 12 |
| 15 | Umsetzungsprodukt gemäss Beispiel 4 | 0,5 | 9,2 | 16 | 11 | 16 | 10 |
| 16 | Umsetzungsprodukt gemäss Beispiel 6 | 0,5 | 8,5 | 21 | 12 | 19 | 10 |
| 17 | Umsetzungsprodukt gemäss Beispiel 7 | 0,5 | 9,1 | 67 | 19 | 53 | 19 |

Tabelle I (Fortsetzung)

| Beispiel Nr. | Leimungsmittel | Einsatzmenge (%) | pH-Wert der Suspension | WA $Cobb_{30}$ ($g/m^2$) | | | |
|---|---|---|---|---|---|---|---|
| | | | | nach Trocknung | | nach 1 Tag Lagerung | |
| | | | | SS | OS | SS | OS |
| 18 | Umsetungsprodukt gemäss Beispiel 8 | 0,5 | 8,1 | 58 | 29 | 56 | 31 |
| 19 | Umsetzungsprodukt gemäss Beispiel 9 | 0,5 | 7,5 | 18 | 14 | 17 | 13 |
| 20 | Umsetzungsprodukt gemäss Beispiel 10 | 0,5 | 7,9 | 15 | 12 | 14 | 11 |

Beispiele 21 bis 25: Man verfährt wie in Beispielen 11 bis 20 angegeben, gibt jedoch das Leimungsmittel und das Retentionsmittel separat zur Faserstoffsuspension, wobei 7%, 14,5% oder 15% Leimungsmittel entweder in geschmolzenem Zustand bei 80°C in Gegenwart von Wasser oder in Pulverform bei Raumtemperatur (15 bis 25°C) in Gegenwart von Wasser und Glasperlen mit einer wässrigen 5%igen Ammoniaklösung zu einer selbstemulgierenden, ebenfalls giessbaren, homogegen und lagerstabilen Emulsion verrührt werden und wobei die in der nachfolgenden Tabelle II angegebenen Formulierungen des Leimungsmittels entstehen. Die angegebenen Val% bedeuten die Anzahl Aequivalente an Ammoniak für 100 Aequivalente, bezogen auf die Anzahl vorhandener, acider Imingruppen der jeweils eingesetzten Leimungsmittel. 10 Sekunden nach der Zugabe der angegebenen Einsatzmenge an Trockensubstanz des Leimungsmittels wird die Faserstoffsuspension mit jeweils der angegebenen Einsatzmenge an Trockensubstanz POLIMIN P® als Retentionsmittel versetzt, wobei sich die Einsatzmengen an Leimungs- und Retentionsmittel auf den Feststoffgehalt der Faserstoffsuspension beziehen. Die Leimungsergebnisse sind ebenfalls aus der Tabelle II zu entnehmen.

Tabelle II

| Beispiel Nr. | Leimungsmittel Formulierung | Einsatzmenge Leimungsmittel(%) | Einsatzmenge Retentionsmittel (%) | Füllmittel | pH-Wert der Suspension | WA $Cobb_{30}$ $(g/m^2)$ nach Trocknung SS | nach Trocknung OS | nach 1 Tag Lagerung OS | nach 1 Tag Lagerung SS |
|---|---|---|---|---|---|---|---|---|---|
| 21 | 15% Umsetzungsprodukt gemäss Beispiel 1 in geschmolzenem Zustand 100 Val% Ammoniak | 0,4 | 0,2 | 20% Kaolin | 5,5 | 22 | 17 | 67 | 58 |
| 22 | 1,5% Umsetzungsprodukt gemäss Beispiel 1 in ge-Pulverform 100 Val% Ammoniak | 0,4 | 0,2 | 20% Kaolin | 5,5 | 25 | 17 | 45 | 20 |
| 23* | 14,5% Umsetzungsprodukt Beispiel 1 in Pulverform 100 Val% Ammoniak | 0,4 | 0,2 | 20% Kaolin | 5,5 | 20 | 15 | 27 | 13 |
| 24 | 7% Umsetzungsprodukt gemäss Beispiel 4 in geschmolzenem Zustand 100 Val% Ammoniak | 0,5 | 0,25 | 20% Kreide | 8,5 | 68 | 59 | 53 | 35 |
| 25 | 7% Umsetzungsprodukt gemäss Beispiel 5 in geschmolzenem Zustand 100 Val% Ammoniak | 1,0 | 0,25 | 20% Kreide | 9,1 | 81 | 72 | 71 | 60 |

* In Beispiel 23 wird das Retentionsmittel zuerst und dann das Leimungsmittel 10 Sekunden nach der Retentionsmittelzugabe zur Faserstoffsuspension gegeben.

Beispiele 26 bis 29: Man verfährt wie in Beispielen 11 bis 20 angegeben, setzt jedoch die in der nachfolgenden Tabelle III angegebenen Füllmittel ein und gibt das Leimungsmittel und das Retentionsmittel separat zur Faserstoffsuspension, wobei 14% Leimungsmittel in Pulverform mit einer wässrigen, 5%igen Ammoniaklösung in Gegenwart von Wasser und Glasperlen verrührt wird und selbstemulgierende, ebenfalls homogene und lagerstabile, in der nachfolgenden Tabelle III angegebene Formulierungen des Leimungsmittels entstehen. Die angegebenen Val% bedeuten die Anzahl Aequivalente an Ammoniak für 100 Aequivalente, bezogen auf die Anzahl vorhandener, acider Imingruppen der jeweils eingesetzten Leimungsmittel. 10 Sekunden nach der Zugabe von 0,4% an Trockensubstanz des Leimungsmittels wird die Faserstoffsuspension mit jeweils 0,2% an Trockensubstanz POLYMIN P® als Retentionsmittel versetzt. Die Einsatzmengen an Füll-, Leimungs- und Retentionsmittel beziehen sich auf den Feststoffgehalt der Faserstoffsuspension. Dies gilt auch für die Alaunmenge. Die Leimungsergebnisse sind ebenfalls aus der Tabelle III zu entnehmen.

Tabelle III

| Beispiel Nr. | Leimungsmittel Formulierung | Füllmittel | pH-Wert der Suspension | WA Cobb$_{30}$ (g/m$^2$) | | | | | |
|---|---|---|---|---|---|---|---|---|---|
| | | | | nach Trocknung | | nach 1 Tag Lagerung | | nach 2 Wochen Lagerung | |
| | | | | SS | OS | SS | OS | SS | OS |
| 26 | 14% Umsetzungsprodukt gemäss Beispiel 1 100 Val% Ammoniak | 20% Kreide (in Gegenwart von 1,5 g $Na_2SO_4$/1 Flotte) | 8,5 | 24 | 15 | 20 | 14 | 32 | 15 |
| 27 | 14% Umsetzungsprodukt gemäss Beispiel 1 100 Val% Ammoniak | 20% Kreide (in Gegenwart von 1,5% $Al_2(SO_4)_3$) | 8,0 | 17 | 15 | 18 | 13 | 23 | 15 |
| 28 | 14% Umsetzungsprodukt gemäss Beispiel 1 100 Val% Ammoniak | 20% Kaolin | 5,5 | 17 | 14 | 17 | 14 | 84 | 42 |
| 29* | 15% Umsetzungsprodukt gemäss Beispiel 1 100 Val% Ammoniak | -* | 6,5 | 19 | 16 | 16 | 13 | 28 | 19 |

* In Beispiel 29 wird eine Faserstoffsuspension eingesetzt, die gebleichten Birkensulfatzellstoff, gebleichten Kiefersulfatzellstoff, Holzschliff im Gewichtsverhältnis 1:1:2, jedoch kein Füllmittel enthält.

Patentansprüche

1. Verfahren zum Leimen von Papier oder Karton, dadurch gekennzeichnet, dass man zu wässrigen, cellulosehaltigen, gegebenenfalls füllmittelhaltigen Faserstoffsuspensionen mindestens
(A) ein Leimungsmittel, welches mindestens eine anionische oder acide, gegebenenfalls in Salzform vorliegende Gruppe und mindestens zwei hydrophobe Substituenten mit jeweils mindestens 5 Kohlenstoffatomen enthält, wobei mindestens einer der hydrophoben Substituenten mindestens 8 Kohlenstoffatome aufweist und mindestens zwei der nächst benachbarten hydrophoben Substituenten miteinander über ein Verbindungsglied verknüpft sind, das mindestens 1 Kohlenstoffatom und 2 Heteroatome, wobei mindestens ein Schwefelatom als Heteroatom vorhanden ist, enthält, und
(B) ein polymeres, kationisches Retentionsmittel
in beliebiger Reihenfolge oder gleichzeitig hinzugibt.

2. Verfahren nach Anspruch 1, dadurch gekennzeichnet, dass man als Komponente (A) ein Leimungsmittel einsetzt, das 1 oder 2 acide Imingruppen und 2 bis 5 hydrophobe Substituenten mit jeweils 6 bis 22 Kohlenstoffatomen enthält, wobei mindestens zwei der nächst benachbarten hydrophoben Substituenten über Verbindungsglieder mit je 1 bis 15 Kohlenstoffatomen, 1 oder 2 Schwefelatomen, 1 bis 5 Stickstoffatomen und gegebenenfalls 1 oder 2 Sauerstoffatomen verknüpft sind und die acide Imingruppe als zweiwertiger Rest $-CO-NH-SO_2-$ oder $-SO_2-NH-SO_2$ vorliegt.

3. Verfahren nach einem der Ansprüche 1 oder 2, dadurch gekennzeichnet, dass man als Komponente (A) ein Leimungsmittel einsetzt, welches durch Umsetzung von mindestens
($a_1$) 1 Mol Chlorsulfonylisocyanat mit
($b_1$) 1 Mol eines Fettalkohols und hierauf mit
($b_2$) 1 Mol eines aromatischen Monoamins, eines primären oder sekundären Fettamins, eines Alkylendiamins oder eines Polyalkylenpolyamins, oder von

($a_1$) 1 Mol Chlorsulfonylisocyanat mit
($b_2$) 2 Mol eines primären oder sekundären Fettamins, oder von
($a_2$) 1 Mol eines unsubstituierten oder mit Halogen oder $C_1$-$C_4$-Alkyl oder -Alkoxy substituierten Diamino-diphenyl-disulfimids mit
($b_3$) 2 Mol eines Fettsäurehalogenids und/oder Alkyl- oder Alkenylisocyanats erhältlich ist.

4. Verfahren nach Anspruch 3, dadurch gekennzeichnet, dass man als Komponente ($b_1$) einen Fettalkohol mit 8 bis 22 Kohlenstoffatomen einsetzt.

5. Verfahren nach Anspruch 3, dadurch gekennzeichnet, dass man als Komponente ($b_2$) ein Mono- oder Dialkylamin, ein Mono- oder Dialkenylamin mit je 8 bis 22 Kohlenstoffatomen im Alkyl- oder Alkenylrest, Mesidin, ein Amino-dimethyl-benzol, Anilin, ein Toluidin oder ein Di- oder Polyamin der Formel

$$H_2N-A_1-[NH-A_1']_{y-1}-NH_2$$

einsetzt, worin $A_1$ und $A_1'$ je Propylen oder Aethylen und y eine ganze Zahl von 1 bis 5 bedeuten.

6. Verfahren nach Anspruch 3, dadurch gekennzeichnet, dass man als Komponente ($b_3$) das Bromid oder Chlorid einer Fettsäure oder das Isocyanat eines Fettamins mit 8 bis 22 Kohlenstoffatomen einsetzt.

7. Verfahren nach einem der Ansprüche 1 bis 6, dadurch gekennzeichnet, dass das eingesetzte Leimungsmittel (A) eine Säurezahl von 15 bis 150 und ein Molekulargewicht von 400 bis 3000 aufweist.

8. Verfahren nach einem der Ansprüche 1 bis 7, dadurch gekennzeichnet, dass das eingesetzte Retentionsmittel (B) ein Molekulargewicht von 1000 bis 2 000 000 aufweist.

9. Verfahren nach Anspruch 8, dadurch gekennzeichnet, dass man als Retentionsmittel (B) ein Polyalkylenimin, Epihalogenhydrin-Addukte von Umsetzungsprodukten aus Polyalkylenpolyaminen und aus aliphatischen Dicarbonsäuren oder von Umsetzungsprodukten aus Polyalkylenpolyaminen, aus Dicyandiamid und gegebenenfalls aus unveresterten oder mit Alkanolen veresterten, organischen Dicarbonsäuren, Umsetzungsprodukte aus Dicyandiamid, aus Formaldehyd, aus Ammoniumsalzen starker anorganischer Säuren und aus Alkylendiaminen oder Polyalkylenpolyaminen, kationisch modifizierte Stärken oder Kohlenhydrate aus Johannisbrot- oder Guarkernmehl, Copolymerisate auf Basis von Polyamid-Aminen oder Umsetzungsprodukte aus Epihalogenhydrinen und aus polymerisierten Diallylaminen einsetzt.

10. Verfahren nach einem der Ansprüche 1 bis 9, dadurch gekennzeichnet, dass man 0,02 bis 3 Gewichtsprozent des Leimungsmittels (A) und 0,02 bis 3 Gewichtsprozent des Retentionsmittels (B), bezogen jeweils auf Trockensubstanz an (A) und (B) und auf den Feststoffgehalt der Faserstoffsuspension, einsetzt.

11. Verfahren nach einem der Ansprüche 1 bis 10, dadurch gekennzeichnet, dass man als fakultative Füllmittel Kondensationsprodukte aus Formaldehyd und Harnstoff, Titandioxyd, Talk, Kaolin, Montmorillonit oder Kreide einsetzt.

12. Wässrige Zusammensetzung zur Durchführung des Verfahrens gemäss einem der Ansprüche 1 bis 11, dadurch gekennzeichnet, dass sie das als mindestens teilweise in Form von Salzen vorliegende Leimungsmittel (A) und gegebenenfalls übliche Zusätze enthält.

13. Wässrige Zusammensetzung zur Durchführung des Verfahrens gemäss einem der Ansprüche 1 bis 11, worin das Leimungsmittel (A) und das Retentionsmittel (B) gleichzeitig zur Faserstoffsuspension gegeben werden, dadurch gekennzeichnet, dass sie

(A) 2 bis 40 Gewichtsprozent Leimungsmittel,

(B) 0,1 bis 20 Gewichtsprozent Retentionsmittel,

bezogen auf Trockensubstanz von (A) und (B) und auf das Gewicht der wässrigen Zusammensetzung, und gegebenenfalls übliche Zusätze enthält.

14. Nach dem Verfahren gemäss einem der Ansprüche 1 bis 11 geleimtes Papier oder geleimter Karton.

15. Verwendung der Komponente (A) gemäss einem der Ansprüche 1 bis 7 zum Leimen von Papier oder Karton.

16. Verbindungen der Formel

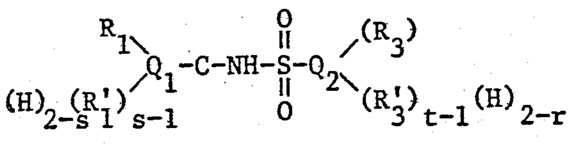 oder

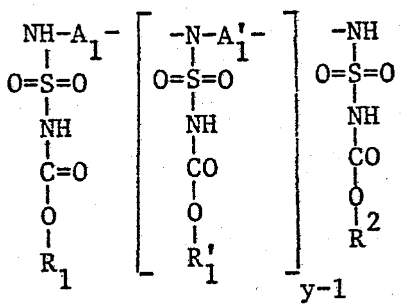 oder

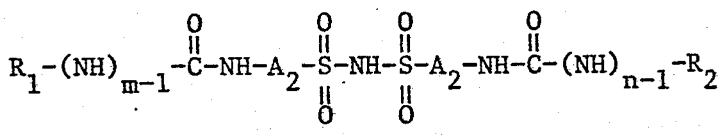 ,

worin $A_1$ und $A_1'$ je Aethylen oder Propylen, $A_2$ unsubstituiertes oder mit Halogen, $C_1$-$C_4$-Alkyl oder -Alkoxy substituiertes Phenyl, $Q_1$ -O-, -NH- oder N<, $Q_2$ -NH- oder N<, $R_1$, $R_1'$ und $R_2$ je Alkyl oder Alkenyl mit 6 bis 22 Kohlenstoffatomen, $R_3$ die für $R_1$ und $R_2$ angegebenen Bedeutungen hat oder je unsubstituiertes oder durch Methyl substituiertes Phenyl, y eine ganze Zahl von 1 bis 5, und m, n, s und t je 1 oder 2 bedeuten, wobei s und t 2 sind, sofern $Q_1$ und $Q_2$ für -N< stehen.

17. Verfahren zur Herstellung der Verbindungen gemäss Anspruch 16, dadurch gekennzeichnet, dass man

($a_1$) 1 Mol Chlorsulfonylisocyanurat mit

($b_1$) 1 Mol eines Fettalkoholes der Formel

$$R_1 - OH ,$$

worin $R_1$ die in Anspruch 16 angegebenen Bedeutungen hat, und hierauf mit

($b_2$) 1 Mol eines primären oder sekundären Fettamins der Formel

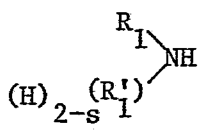 oder 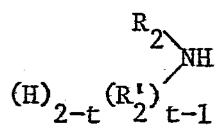

worin s und t je 1 oder 2 sind, $R_1$, $R_1'$ und $R_2$ die in Anspruch 16 angegebenen Bedeutungen haben, $R_2'$ die für $R_2$ angegebenen Bedeutungen hat, und $R_2$ und $R_2'$ voneinander verschieden oder gleich sind, oder eines aromatischen Monoamins der Formel

$$R_4 - NH_2 ,$$

worin $R_4$ unsubstituiertes oder durch Methyl substituiertes Phenyl bedeutet, oder eines Alkylendiamins oder Polyalkylenpolyamins der Formel

$$H_2N-A_1-[NH-A_1']_{y-1}-NH_2 ,$$

worin $A_1$ und $A_1'$ je Propylen oder Aethylen und y eine ganze Zahl von 1 bis 5 bedeuten, oder

($a_1$) 1 Mol Chlorsulfonylisocyanat mit

($b_2$) 2 Mol eines primären oder sekundären Fettamins der Formel

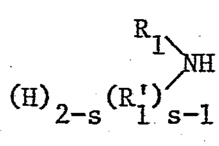 oder 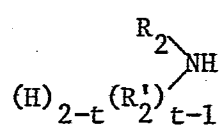 ,

worin $R_1$, $R_1'$, $R_2$, $R_2'$ und s und t jeweils die angegebenen Bedeutungen haben, oder

($a_2$) 1 Mol eines Diamino-diphenyl-disulfimids der Formel

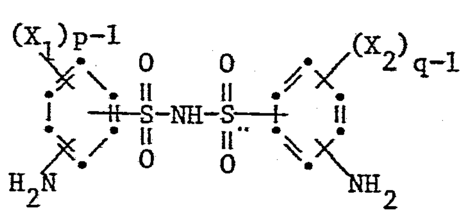 ,

worin $X_1$ Halogen oder Alkyl oder Alkoxy mit je 1 bis 4 Kohlenstoffatomen, $X_2$ die für $X_1$ angegebenen Bedeutungen hat und $X_1$ und $X_2$ gleich oder voneinander verschieden sind, und p und q je 1 oder 2 sind, mit

($b_3$) 2 Mol eines Fettsäurehalogenids der Formel

$$R_1-\overset{O}{\overset{\|}{C}}-Z_1 \qquad \text{oder}$$

$$R_2-\overset{O}{\overset{\|}{C}}-Z_2$$

oder eines Alkyl- oder Alkenylisocyanates der Formel

$R_1$-N=C=O oder

$R_2$-N=C=O oder deren Gemische ,

worin $Z_1$ und $Z_2$ je Halogen bedeuten und $R_1$ und $R_2$ die in Anspruch 16 angegebenen Bedeutungen haben,
miteinander umsetzt.

FO 7.1/PLP/gs*/sch*/jt*